# EUROPEAN PATENT SPECIFICATION

(11) **EP 3 939 379 B1**
(45) Date of publication and mention of the grant of the patent: **30.09.2026**
(21) Application number: 20712232.6
(22) Date of filing: 09.03.2020
(51) Int. Cl.: H05B 6/10, A24F 47/00

(54) **AEROSOL PROVISION DEVICE**
AEROSOLBEREITSTELLUNGSVORRICHTUNG
DISPOSITIF DE FOURNITURE D'AÉROSOL

(30) Priority: 11.03.2019 GB 201903249
(43) Date of publication of application: 19.01.2022
(60) Divisional application: 23152265.7 / 4 188 036; 26158642.4 / 4 717 104
(73) Proprietor: Nicoventures Trading Limited, London Greater London WC2R 3LA (GB)
(72) Inventor: SAYED, Ashley John, London Greater London WC2R 3LA (GB); WARREN, Luke James, London Greater London WC2R 3LA (GB); WOODMAN, Thomas Alexander John, London Greater London WC2R 3LA (GB)
(74) Representative: Dehns
(86) International application number: PCT/EP2020/056238
(87) International publication number: WO 2020/182747

(56) References cited:
- WO-A1-2018/073376
- US-A1- 2004 149 297
- US-A1- 2016 338 412
- US-A1- 2017 055 583
- US-A1- 2017 055 585
- US-A1- 2017 112 196
- US-A1- 2017 156 399
- US-A1- 2018 154 103
- US-A1- 2019 069 605

## Description

### Technical Field

The present invention relates to aerosol provision devices and methods of operating aerosol provision devices.

### Background

Smoking articles such as cigarettes, cigars and the like burn tobacco during use to create tobacco smoke. Attempts have been made to provide alternatives to these articles that burn tobacco by creating products that release compounds without burning. Examples of such products are heating devices which release compounds by heating, but not burning, the material. The material may be for example tobacco or other non-tobacco products, which may or may not contain nicotine.

US 2017/0055585 A1 discloses an inductive heating device for heating an aerosol forming substrate comprising a susceptor.

### Summary

According to an aspect of the present invention, there is provided an aerosol provision device as recited in claim 1.
Further optional features are recited in each of claims 2 to 15.

Further features and advantages of the invention will become apparent from the following description of preferred embodiments of the invention, given by way of example only, which is made with reference to the accompanying drawings.

### Brief Description of the Drawings

Figure 1 shows a front view of an example of an aerosol provision device;
Figure 2 shows a front view of the aerosol provision device of Figure 1 with an outer cover removed;
Figure 3 shows a cross-sectional view of the aerosol provision device of Figure 1;
Figure 4 shows an exploded view of the aerosol provision device of Figure 2;
Figure 5A shows a cross-sectional view of a heating assembly within an aerosol provision device;
Figure 5B shows a close-up view of a portion of the heating assembly of Figure 5A;
Figure 6 shows a front view of the device;
Figure 7 shows a perspective view of the housing of the device;
Figure 8 shows a perspective view of the device without the housing;
Figure 9 depicts a perspective view of LEDs arranged within the device;
Figure 10 shows an outer member comprising a plurality of apertures;
Figure 11 shows components of the device arranged above the LEDs;
Figure 12 shows a system comprising a controller, a heater assembly, an input interface and an indicator assembly;
Figures 13A-D show the outer member illuminated by a plurality of LEDs;
Figure 14 shows a flow diagram of a method of operating a device; and
Figure 15 shows a flow diagram of a method of operating a device.

### Detailed Description

As used herein, the term "aerosol generating material" includes materials that provide volatilised components upon heating, typically in the form of an aerosol. Aerosol generating material includes any tobacco-containing material and may, for example, include one or more of tobacco, tobacco derivatives, expanded tobacco, reconstituted tobacco or tobacco substitutes. Aerosol generating material also may include other, non-tobacco, products, which, depending on the product, may or may not contain nicotine. Aerosol generating material may for example be in the form of a solid, a liquid, a gel, a wax or the like. Aerosol generating material may for example also be a combination or a blend of materials. Aerosol generating material may also be known as "smokable material".

Apparatus is known that heats aerosol generating material to volatilise at least one component of the aerosol generating material, typically to form an aerosol which can be inhaled, without burning or combusting the aerosol generating material. Such apparatus is sometimes described as an "aerosol generating device", an "aerosol provision device", a "heat-not-burn device", a "tobacco heating product device" or a "tobacco heating device" or similar. Similarly, there are also so-called e-cigarette devices, which typically vaporise an aerosol generating material in the form of a liquid, which may or may not contain nicotine. The aerosol generating material may be in the form of or be provided as part of a rod, cartridge or cassette or the like which can be inserted into the apparatus. A heater for heating and volatilising the aerosol generating material may be provided as a "permanent" part of the apparatus.

An aerosol provision device can receive an article comprising aerosol generating material for heating. An "article" in this context is a component that includes or contains in use the aerosol generating material, which is heated to volatilise the aerosol generating material, and optionally other components in use. A user may insert the article into the aerosol provision device before it is heated to produce an aerosol, which the user subsequently inhales. The article may be, for example, of a predetermined or specific size that is configured to be placed within a heating chamber of the device which is sized to receive the article.

A first aspect of the present disclosure defines an aerosol provision device comprising an indicator assembly configured to provide an indication that the device is ready for use within a predetermined period of time after causing a coil, such as an inductor coil, to heat a heater component, such as a susceptor. As will be discussed in more detail herein, a susceptor is an electrically conducting object, which is heatable by penetration with a varying magnetic field. An inductor coil generates the varying magnetic field which causes the susceptor to be heated. Once heated, the susceptor transfers heat to aerosol generating material, which releases an aerosol. In one example, the susceptor defines a receptacle and the susceptor receives the aerosol generating material.

It has been found that certain heating systems are able to heat aerosol generating material to a suitable temperature within a reduced period of time when compared to other types of heating assemblies. Accordingly, a user of the device is able to draw on the device to inhale the aerosol in a period of less than about 60 seconds. To ensure that the user is aware the device is ready for use, the aerosol provision device comprises an indicator assembly to indicate that the device is ready for the user to inhale the aerosol. Because the inductor coil is able to heat the susceptor and aerosol generating material quickly, the aerosol generating material will have released a sufficient amount of aerosol at the time the device indicates that the device is ready.

"Ready for use" may mean that the aerosol generating material has reached a desired/sufficient temperature, or may mean that the aerosol generating material has generated a desired/sufficient volume of aerosol, or may mean that the user can take a first "puff" on the device, to inhale aerosol generated by the aerosol generating material.

The reference to "within a predetermined period time" includes examples where the indicator provides an indication at a predetermined period of time. For example, the characteristics of the article in use with the aerosol provision device and the heating applied by the aerosol provision device may be known, so that the time to be "ready for use" can be predetermined. It also includes examples where some characteristics of the aerosol provision device and/or the article are monitored to determine whether the article is ready for use. For example, a temperature sensor measuring at or above a predetermined temperature may indicate that the device is ready for use.

In some examples, the predetermined period of time is less than about 50 seconds, less than about 40 seconds, less than about 30 seconds, less than about 20 seconds, less than about 15 seconds, or less than about 10 seconds after causing the coil to heat the heater component. Inductive heating systems may be more efficient at heating the aerosol generating material. In inductive heating systems, the predetermined period of time may be less than about 20 seconds.

In some examples the coil is an inductor coil and the heater component is a susceptor. The controller may be configured to cause the coil to heat the heater component by causing the inductor coil to generate a varying magnetic field. The susceptor is heatable by penetration with the varying magnetic field.

The device may be configured to operate in one of a first mode and a second mode, and when the device is operated in the second mode the inductor coil is configured to cause the aerosol generating material to be heated to a higher temperature than when the device is operated in the first mode, and wherein the predetermined period of time is a first predetermined time when the device is operating in the first mode and a second predetermined time when the device is operating in the second mode, the second predetermined time being different than the first predetermined time.

Thus, in some examples the device can be operated in different modes. In the first mode for example, the inductor coil may be configured to heat the susceptor to a first temperature. In the second mode, for example, the inductor coil may be configured to heat the susceptor to a second temperature. In some examples the second temperature is greater than the first temperature. The first and second temperatures may be average temperatures of the susceptor measured during a single session.

The first temperature may be between about 240°C and about 260°C and the second temperature may be between about 270°C and about 290°C. The temperature of the aerosol generating material may be marginally less than the temperature of the susceptor.

The first mode may be known as a default mode, and the second mode may be known as a boost mode. The second mode may, for example, generate a higher volume or concentration of aerosol than the first mode.

In a particular example, the second predetermined time is less than the first predetermined time. For example, if the aerosol generating material is heated to a higher temperature, it may release aerosol quicker than if was heated to a lower temperature. This can mean that the device is ready for use quicker.

In another example, the device is configured to operate in one of a first mode and a second mode, and when the device is operated in the second mode the inductor coil is configured to cause the susceptor to be heated to a higher temperature than when the device is operated in the first mode, and wherein the predetermined period of time is a first predetermined time when the device is operating in the first mode and a second predetermined time when the device is operating in the second mode, the second predetermined time being less than the first predetermined time.

In some examples, the indicator assembly may indicate the selected heating mode. In some examples this indication is the same indication as that which indicates the device is ready for use. Thus the type of indication used to indicate that the device is ready for use may be based on the mode in which the device is operating. In other examples the indication that indicates the mode may occur after a particular mode is selected, but before the device is ready for use. Thus, two separate indications may occur. A first indication may indicate the mode, and a second indication may indicate that the device is ready for use. This can allow the user to cancel the heating if they accidentally select the wrong mode. In a particular example the first indication is provided by a haptic component, and the second indication is provided by a visual component. This is useful because the user may be holding the device when they select the heating mode, but may place the device on a surface as they wait for the device to be ready for use. The visual indication can be more easily seen if the user is no longer holding the device.

The device may further comprise an input interface, and the controller is configured to cause the inductor coil to generate the varying magnetic field in response to an input received from the input interface. Thus, a user can interact with, or operate the input interface to cause the device to begin heating.

The input interface may also be referred to as a user interface. The input interface may be a button, touch screen, dial, knob, or a wireless connection to a mobile device (for example using Bluetooth).

The input interface comprises a button. The input comprises a signal indicating the button has been released. Thus, the inductor coil begins heating the susceptor only once the button has been released. While the user is holding down the button, the inductor coil may not heat the susceptor. The predetermined period of time therefore initiates when a user releases the button. The button may be a software button or a hardware button. The signal may be a single signal, or may be two or more signals.

In a particular example, the input further comprises a signal indicating a length of time that the button has been pressed and the controller is configured to cause the inductor coil to generate the varying magnetic field in response to (i) receiving the signal indicating that the button has been released, and (ii) determining that the length of time that the button has been pressed is greater than or equal to a threshold time period. The signal indicating the length of time that the button has been pressed may be part of the same signal which indicates that the button has been released, or may be a separate signal. Thus, in some examples, the inductor coil may only begin heating the susceptor if the button is pressed for a certain length of time that is greater than or equal to a threshold time period. In a particular example, the threshold time period is 3 seconds or 5 seconds. If the button is held and released for less than the threshold time period, the inductor coil may not begin heating the susceptor. This can avoid heating the susceptor if the user accidentally presses of the button, which can waste energy. Thus, if the controller determines that the length of time that the button has been pressed is less than the threshold, the controller determines not to cause the inductor coil to generate the varying magnetic field.

In one example, the device is configured to operate in a first mode if the length of time that the button has been pressed is greater than or equal to a first threshold time period and is less than a second threshold time period, and the device is configured to operate in a second mode if the length of time that the button has been pressed is greater than or equal to the second threshold time period. The first threshold time period may be 3 seconds, and the second threshold time period may be 5 seconds, for example. Thus, using a single button the user can select different modes. Having a single interface to select multiple modes can simplify operation of the device and reduce the number of components. A reduced number of components can make the device more lightweight and there are fewer parts to break or stop functioning.

The device may comprise a puff detector for detecting when a user takes a puff on the device, and the controller is configured to cause the inductor coil to generate the varying magnetic field in response to the puff detector detecting when the user takes a puff on the device. Thus, the device may begin heating automatically when a user inhales on the device.

In some examples the indicator assembly provides an indication that the inductor coil has begun to generate the varying magnetic field. This can avoid the user trying to start operation of the device again.

The indicator assembly comprises a visual component configured to provide a visual indication that the device is ready for use. For example, the visual component may comprise an LED, a plurality of LEDs, a display, an eInk display, or a mechanical element which moves to display one or more patterns, for example. In some examples, the visual component is configured to emit light.

In a particular example, the indicator assembly comprises a plurality of LEDs, and the number of illuminated LEDs indicates when the device is ready for use. For example, when the inductor coil first begins to generate the varying magnetic field there may be a first number of LEDs illuminated and when the device is ready for use there may be a second number of LEDs illuminated, where the second number is greater than the first number. The first number of LEDs may be zero. The second number may be all of the LEDs. The indicator assembly may therefore indicate how close the device is to being ready for use. The LEDs may be sequentially illuminated during the predetermined period of time.

In a particular example there are four LEDs, and the LEDs are sequentially illuminated during the predetermined period of time. For example, the first LED may be illuminated 5 seconds after causing the inductor coil to generate the magnetic field, the second LED may be illuminated 10 seconds after causing the inductor coil to generate the magnetic field, the third LED may be illuminated 15 seconds after causing the inductor coil to generate the magnetic field and the fourth LED may be illuminated 20 seconds after causing the inductor coil to generate the magnetic field. Illumination of the final LED may indicate that the device is ready for use. The earlier illuminated LEDs may remain illuminated as the next LED is illuminated. Alternatively, as the subsequent LEDs is illuminated, the earlier LED may switch off.

The indicator assembly comprises a haptic component configured to provide haptic feedback to indicate that the device is ready for use. For example, the haptic component may be a haptic motor which causes the device to vibrate when the device is ready for use. In some examples the haptic component provides haptic feedback according to a first pattern after the inductor coil generates the varying magnetic field and provides haptic feedback according to a second pattern when the device is ready for use. The first pattern may last until the device is ready for use or may terminate after a short time. Accordingly, the haptic component may also indicate that the device has begun heating the aerosol generating material so that the user is aware the device is operating.

In another example, the indicator assembly comprises an audible indicator configured to emit sound to indicate that the device is ready for use. The audible indicator may be a transducer, buzzer, beeper, etc.

The indicator assembly comprises a haptic component and a visual component. The haptic component may be configured to provide a haptic indication that the inductor coil has begun to generate the varying magnetic field. The visual component may be configured to provide a visual indication that the device is ready for use.

In some examples the indicator assembly is configured to indicate the time left until the device finishes operating. For example, the indicator assembly may provide different indications depending upon the time left until the device finishes operating. The device may "finish operating" at the time the inductor coil has ceased generating the varying magnetic field, or at the time the aerosol temperature/volume is considered to fall below an acceptable level, which may be several seconds after point at which the inductor coil has ceased generating the magnetic field.

In a particular example, the indicator assembly comprises a plurality of LEDs, and the number of illuminated LEDs indicates the time left until the device finishes operating. For example, when the device is operating there may be a first number of LEDs illuminated and when the device has finished operating there may be a second number of LEDs illuminated, where the second number is less than the first number. The second number may be zero, for example. The first number may be all of the LEDs. The LEDs may therefore "count down" as the device gets closer to finishing.

In a particular example there are a plurality of LEDs, such as four LEDs, and the LEDs are sequentially switched off as the end of the heating session approaches. For example, all four LEDs may be illuminated 20 seconds before the device finishes operating. When only 15 seconds remain, one of the four LEDs may be switched off. When only 10 seconds remain, another LED may be switched off. When only 5 seconds remain another LED may be switched off, and when there are 0 seconds remaining all four LEDs may be switched off.

The haptic component provides different haptic feedback patterns depending upon the time left. For example, the haptic component may provide haptic feedback to indicate that is a certain period of time remaining. The type of haptic feedback may be indicative of how much time is remaining. For example, when there are 20 seconds remaining, there may be a short, low intensity haptic feedback and when there are 5 seconds or 0 seconds remaining, the haptic feedback may be longer and more intense.

In a further example, the audible indicator may provide different sounds depending upon the time left. For example, the pitch, tone, sound pattern, etc. may change over time.

In another example, the controller is configured to cause the indicator assembly to indicate that the device has finished operating or is about to finish operating within a third predetermined period of time after causing the inductor coil to generate the varying magnetic field. Thus, the indicator assembly may indicate the moment at which it finishes operating, or is about to finish operating. For example, the when the device finishes operating the visual indicator may no longer provide any visual indication. In a particular example, all of the LEDs may be switched off when the device has finished operating or is about to finish operating. This indicates to the user that they should cease inhaling from the device. The third predetermined time is longer than first or second predetermined times described above. The third predetermined time may be three minutes, three minutes and thirty seconds, or four minutes for example. The third predetermined time may depend upon the mode in which the device is operating.

The device may define a longitudinal axis. In some examples, the inductor coil is a first inductor coil, and the device further comprises a second inductor coil for generating a second varying magnetic field. In a particular arrangement, the first inductor coil is adjacent the second inductor coil in a direction along the longitudinal axis and the controller is configured to cause the second inductor coil to generate the second varying magnetic field after causing the indicator assembly to indicate that the device is ready for use. In use, the aerosol is drawn along a flow path of the device towards a proximal end of the device, and the first inductor coil is arranged closer to the proximal end of the device than the second inductor coil.

Accordingly, the device may comprise two inductor coils, where the first inductor coil is closer to a mouth end of the device. The first inductor coil therefore heats aerosol generating material which is closer to the mouth of the user. Initially the first inductor coil is operated. The second inductor coil can be operated at a later time. For example, the controller may cause the second inductor coil to generate the second magnetic field at a fourth predetermined time after causing the first inductor coil to generate the first magnetic field. The fourth predetermined time may be between about 40 seconds and about 60 seconds, for example. The fourth predetermined time may depend upon the mode in which the device is operating.

The first inductor coil may continue to generate the first magnetic field while the second inductor coil is generating the second magnetic field.

In a particular example, the first inductor coil has a first length, second inductor coil has a second length, and the first length is shorter than the second length. A shorter length heats a lower volume of aerosol generating material, which generates a lower volume of aerosol, thereby reducing the phenomenon known as "hot puff".

In another aspect, there is provided a method of operating the aerosol provision device described above. The method comprises causing an inductor coil of the aerosol provision device to generate a varying magnetic field for heating a susceptor, and causing an indicator assembly of the aerosol provision device to indicate that the device is ready for use within a predetermined period of time after causing the inductor coil to generate the varying magnetic field, wherein the predetermined period of time is less than about 20 seconds.

In some examples, the device is configured to operate in one of a first mode and a second mode, and the method comprises operating the device in the second mode by causing the inductor coil to cause the aerosol generating material to be heated to a higher temperature than when the device is operated in the first mode, and wherein the predetermined period of time is a first predetermined time when the device is operating in the first mode and a second predetermined time when the device is operating in the second mode, the second predetermined time being different than the first predetermined time.

In another example, the device is configured to operate in one of a first mode and a second mode, and the method comprises operating the device in the second mode by causing the inductor coil to heat the susceptor to a higher temperature than when the device is operated in the first mode, and wherein the predetermined period of time is a first predetermined time when the device is operating in the first mode and a second predetermined time when the device is operating in the second mode, the second predetermined time being less than the first predetermined time.

The method may further comprise causing the inductor coil to generate the varying magnetic field in response to receiving an input from an input interface of the device.

The input interface may comprise a button, and the input comprises a signal indicating (i) that the button has been released, and (ii) a length of time that the button has been pressed. The method may further comprise causing the inductor coil to generate the varying magnetic field in response to receiving the signal and determining that the length of time that the button has been pressed is greater than or equal to a threshold time period.

The method may further comprise causing the inductor coil to generate the varying magnetic field in response to a puff detector detecting that a user takes a puff on the device.

The method may further comprise causing the indicator assembly to indicate the time left until the device finishes operating.

The method may further comprise causing the indicator assembly to indicate that the device has finished operating or is about to finish operating within a third predetermined period of time after causing the inductor coil to generate the varying magnetic field.

In one arrangement, the inductor coil is a first inductor coil, and the device further comprises a second inductor coil for generating a second varying magnetic field. The first inductor coil may be adjacent the second inductor coil in a direction along the longitudinal axis and in use, the aerosol is drawn along a flow path of the device towards a proximal end of the device, and the first inductor coil is arranged closer to the proximal end of the device than the second inductor coil. The method may further comprise causing the second inductor coil to generate the second varying magnetic field after causing the indicator assembly to indicate that the device is ready for use.

Although this method is described in relation to an inductive heater, it will be appreciated that this method may also be applied to a device with a non-inductive heater assembly. For example, instead of an inductor coil, the device may comprise a heater assembly configured to heat aerosol generating material.

In a second aspect, an aerosol provision device comprises a heater assembly configured to heat aerosol generating material, an indicator assembly, and a controller. The controller is configured to cause the heater assembly to begin heating the aerosol generating material, and cause the indicator assembly to indicate that the device is ready for use within a predetermined period of time, wherein the predetermined period of time is less than about 20 seconds after causing the heater assembly to begin heating the aerosol generating material.

Thus, in some examples, the device may comprise a heater assembly that is not inductive. For example, the heater assembly may comprise resistive heating components configured to heat aerosol generating material. The features described above in relation to the first aspect may be applied to the aerosol provision device of the second aspect.

In the third aspect, an aerosol provision device comprises an inductor coil for generating a varying magnetic field, a susceptor arranged to heat aerosol generating material, wherein the susceptor is heatable by penetration with the varying magnetic field, an indicator assembly, and a controller. The controller is configured to cause the inductor coil to begin generating the varying magnetic field, and cause the indicator assembly to indicate that the device has finished operating or is about to finish operating within a predetermined period of time after causing the inductor coil to begin heating the aerosol generating material. Thus the user can be informed when the device has finished operating or is about to finish operating. This stops the user from continuing to use the device when the aerosol generated may no longer be of sufficient volume, concentration or temperature.

In another aspect, a method of operating an aerosol provision device, comprises causing an inductor coil of the aerosol provision device to generate a varying magnetic field for heating a susceptor and causing an indicator assembly of the aerosol provision device to indicate that the device has finished operating or is about to finish operating within a predetermined period of time after causing the inductor coil assembly to begin heating the aerosol generating material.

Although this method is described in relation to an inductive heater, it will be appreciated that this method may also be applied to a device with a non-inductive heater assembly. For example, instead of an inductor coil, the device may comprise a heater assembly configured to heat aerosol generating material.

In a particular example, the indicator assembly comprises one or more Light Emitting Diodes (LEDs) and an outer member positioned above the one or more LEDs. The outer member comprises a plurality of apertures visible from outside the aerosol provision device. Electromagnetic radiation (in the form of visible light for example) can pass through the plurality of apertures and be viewed by a user. At least a portion of the outer member may form an outer surface of the device.

The indicator assembly may further comprise a light-shaping member positioned between the one or more LED and the outer member. The light shaping member may comprise one or more light pipes to guide light through the light-shaping member to produce a particular pattern or design. The light-shaping member may comprise opaque regions configured to block a portion of the light from the LEDs. The light-shaping member may comprise transparent or translucent regions to allow the light to pass through. The light-shaping member may alternatively comprise openings to allow the light to pass through. A light-shaping member that comprises opaque regions and transparent or translucent regions may be more robust than a light-shaping member with openings. Translucent regions can also additionally diffuse/soften the light.

In some examples, the light shaping member is formed from two or more overmolded components. For example, the opaque and transparent/translucent regions may be formed from two overmolded components.

In one example, the light-shaping member comprises an opaque region extending around the periphery/perimeter/circumference of the light-shaping member. This can prevent light from leaking around the outside of the outer member. The opaque region may be an outer ring.

In one example the opaque region is coloured black or dark grey.

In one example, the opaque region is cross-shaped.

In a specific example, the device comprises four LEDs, wherein each of the four LEDs is located below the light-shaping member and are positioned between adjacent opaque regions such that the light from the LEDs separates into 4 quadrants. The opaque regions are configured to prevent light bleed from one quadrant to the adjacent quadrant.

Preferably, the device is a tobacco heating device, also known as a heat-not-burn device.

As briefly mentioned above, in some examples, the coil(s) is/are configured to, in use, cause heating of at least one electrically-conductive heating component/element (also known as a heater component/element), so that heat energy is conductible from the at least one electrically-conductive heating component to aerosol generating material to thereby cause heating of the aerosol generating material.

In some examples, the coil(s) is/are configured to generate, in use, a varying magnetic field for penetrating at least one heating component/element, to thereby cause induction heating and/or magnetic hysteresis heating of the at least one heating component. In such an arrangement, the or each heating component may be termed a "susceptor". A coil that is configured to generate, in use, a varying magnetic field for penetrating at least one electrically-conductive heating component, to thereby cause induction heating of the at least one electrically-conductive heating component, may be termed an "induction coil" or "inductor coil".

The at least one heating component, for example at least one electrically-conductive heating component, may be included in an article for insertion into a heating zone of the device, wherein the article also comprises the aerosol generating material and is removable from the heating zone after use. Alternatively, both the device and such an article may comprise at least one respective heating component, for example at least one electrically-conductive heating component, and the coil(s) may be to cause heating of the heating component(s) of each of the device and the article when the article is in the heating zone.

In some examples, the coil(s) is/are helical. In some examples, the coil(s) encircles at least a part of a heating zone of the device that is configured to receive aerosol generating material. In some examples, the coil(s) is/are helical coil(s) that encircles at least a part of the heating zone. The heating zone may be a receptacle, shaped to receive the aerosol generating material.

In some examples, the device comprises an electrically-conductive heating component that at least partially surrounds the heating zone, and the coil(s) is/are helical coil(s) that encircles at least a part of the electrically-conductive heating component. In some examples, the electrically-conductive heating component is tubular. In some examples, the coil is an inductor coil.

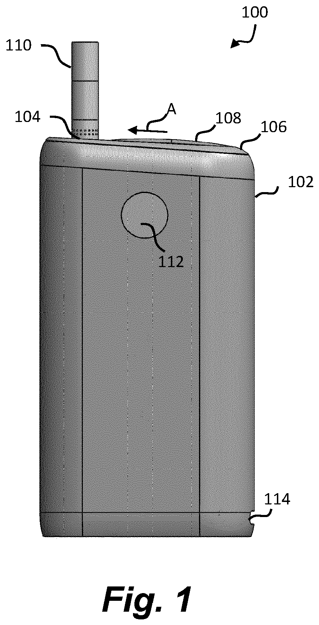

Figure 1 shows an example of an aerosol provision device 100 for generating aerosol from an aerosol generating medium/material. In broad outline, the device 100 may be used to heat a replaceable article 110 comprising the aerosol generating medium, to generate an aerosol or other inhalable medium which is inhaled by a user of the device 100.

The device 100 comprises a housing 102 (in the form of an outer cover) which surrounds and houses various components of the device 100. The device 100 has an opening 104 in one end, through which the article 110 may be inserted for heating by a heating assembly. In use, the article 110 may be fully or partially inserted into the heating assembly where it may be heated by one or more components of the heater assembly.

The device 100 of this example comprises a first end member 106 which comprises a lid 108 which is moveable relative to the first end member 106 to close the opening 104 when no article 110 is in place. In Figure 1, the lid 108 is shown in an open configuration, however the cap 108 may move into a closed configuration. For example, a user may cause the lid 108 to slide in the direction of arrow "A".

The device 100 may also include an input interface 112, which may comprise a button or switch, which operates the device 100 when pressed. For example, a user may turn on the device 100 by operating the input interface 112.

The device 100 may also comprise an electrical connector/component, such as a socket/port 114, which can receive a cable to charge a battery of the device 100. For example, the socket 114 may be a charging port, such as a USB charging port. In some examples the socket 114 may be used additionally or alternatively to transfer data between the device 100 and another device, such as a computing device.

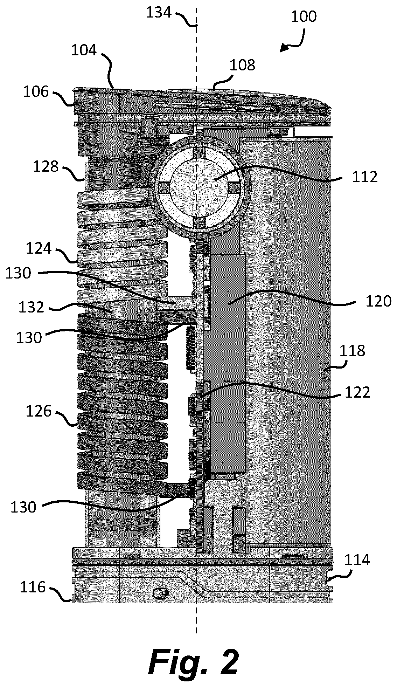

Figure 2 depicts the device 100 of Figure 1 with the outer cover 102 removed and without an article 110 present. The device 100 defines a longitudinal axis 134.

As shown in Figure 2, the first end member 106 is arranged at one end of the device 100 and a second end member 116 is arranged at an opposite end of the device 100. The first and second end members 106, 116 together at least partially define end surfaces of the device 100. For example, the bottom surface of the second end member 116 at least partially defines a bottom surface of the device 100. Edges of the outer cover 102 may also define a portion of the end surfaces. In this example, the lid 108 also defines a portion of a top surface of the device 100.

The end of the device closest to the opening 104 may be known as the proximal end (or mouth end) of the device 100 because, in use, it is closest to the mouth of the user. In use, a user inserts an article 110 into the opening 104, operates the user control 112 to begin heating the aerosol generating material and draws on the aerosol generated in the device. This causes the aerosol to flow through the device 100 along a flow path towards the proximal end of the device 100.

The other end of the device furthest away from the opening 104 may be known as the distal end of the device 100 because, in use, it is the end furthest away from the mouth of the user. As a user draws on the aerosol generated in the device, the aerosol flows away from the distal end of the device 100.

The device 100 further comprises a power source 118. The power source 118 may be, for example, a battery, such as a rechargeable battery or a non-rechargeable battery. Examples of suitable batteries include, for example, a lithium battery (such as a lithium-ion battery), a nickel battery (such as a nickel-cadmium battery), and an alkaline battery. The battery is electrically coupled to the heating assembly to supply electrical power when required and under control of a controller (not shown) to heat the aerosol generating material. In this example, the battery is connected to a central support 120 which holds the battery 118 in place. The central support 120 may also be known as a battery support, or battery carrier.

The device further comprises at least one electronics module 122. The electronics module 122 may comprise, for example, a printed circuit board (PCB). The PCB 122 may support at least one controller, such as a processor, and memory. The PCB 122 may also comprise one or more electrical tracks to electrically connect together various electronic components of the device 100. For example, the battery terminals may be electrically connected to the PCB 122 so that power can be distributed throughout the device 100. The socket 114 may also be electrically coupled to the battery via the electrical tracks.

In the example device 100, the heating assembly is an inductive heating assembly and comprises various components to heat the aerosol generating material of the article 110 via an inductive heating process. Induction heating is a process of heating an electrically conducting object (such as a susceptor) by electromagnetic induction. An induction heating assembly may comprise an inductive element, for example, one or more inductor coils, and a device for passing a varying electric current, such as an alternating electric current, through the inductive element. The varying electric current in the inductive element produces a varying magnetic field. The varying magnetic field penetrates a susceptor suitably positioned with respect to the inductive element, and generates eddy currents inside the susceptor. The susceptor has electrical resistance to the eddy currents, and hence the flow of the eddy currents against this resistance causes the susceptor to be heated by Joule heating. In cases where the susceptor comprises ferromagnetic material such as iron, nickel or cobalt, heat may also be generated by magnetic hysteresis losses in the susceptor, i.e. by the varying orientation of magnetic dipoles in the magnetic material as a result of their alignment with the varying magnetic field. In inductive heating, as compared to heating by conduction for example, heat is generated inside the susceptor, allowing for rapid heating. Further, there need not be any physical contact between the inductive heater and the susceptor, allowing for enhanced freedom in construction and application.

The induction heating assembly of the example device 100 comprises a susceptor arrangement 132 (herein referred to as "a susceptor"), a first inductor coil 124 and a second inductor coil 126. The first and second inductor coils 124, 126 are made from an electrically conducting material. In this example, the first and second inductor coils 124, 126 are made from Litz wire/cable which is wound in a helical fashion to provide helical inductor coils 124, 126. Litz wire comprises a plurality of individual wires which are individually insulated and are twisted together to form a single wire. Litz wires are designed to reduce the skin effect losses in a conductor. In the example device 100, the first and second inductor coils 124, 126 are made from copper Litz wire which has a rectangular cross section. In other examples the Litz wire can have other shape cross sections, such as circular.

The first inductor coil 124 is configured to generate a first varying magnetic field for heating a first section of the susceptor 132 and the second inductor coil 126 is configured to generate a second varying magnetic field for heating a second section of the susceptor 132. In this example, the first inductor coil 124 is adjacent to the second inductor coil 126 in a direction along the longitudinal axis 134 of the device 100 (that is, the first and second inductor coils 124, 126 to not overlap). The susceptor arrangement 132 may comprise a single susceptor, or two or more separate susceptors. Ends 130 of the first and second inductor coils 124, 126 can be connected to the PCB 122.

It will be appreciated that the first and second inductor coils 124, 126, in some examples, may have at least one characteristic different from each other. For example, the first inductor coil 124 may have at least one characteristic different from the second inductor coil 126. More specifically, in one example, the first inductor coil 124 may have a different value of inductance than the second inductor coil 126. In Figure 2, the first and second inductor coils 124, 126 are of different lengths such that the first inductor coil 124 is wound over a smaller section of the susceptor 132 than the second inductor coil 126. Thus, the first inductor coil 124 may comprise a different number of turns than the second inductor coil 126 (assuming that the spacing between individual turns is substantially the same). In yet another example, the first inductor coil 124 may be made from a different material to the second inductor coil 126. In some examples, the first and second inductor coils 124, 126 may be substantially identical.

In this example, the first inductor coil 124 and the second inductor coil 126 are wound in opposite directions. This can be useful when the inductor coils are active at different times. For example, initially, the first inductor coil 124 may be operating to heat a first section of the article 110, and at a later time, the second inductor coil 126 may be operating to heat a second section of the article 110. Winding the coils in opposite directions helps reduce the current induced in the inactive coil when used in conjunction with a particular type of control circuit. In Figure 2, the first inductor coil 124 is a right-hand helix and the second inductor coil 126 is a left-hand helix. However, in another embodiment, the inductor coils 124, 126 may be wound in the same direction, or the first inductor coil 124 may be a left-hand helix and the second inductor coil 126 may be a right-hand helix.

The susceptor 132 of this example is hollow and therefore defines a receptacle within which aerosol generating material is received. For example, the article 110 can be inserted into the susceptor 132. In this example the susceptor 120 is tubular, with a circular cross section.

The device 100 of Figure 2 further comprises an insulating member 128 which may be generally tubular and at least partially surround the susceptor 132. The insulating member 128 may be constructed from any insulating material, such as plastic for example. In this particular example, the insulating member is constructed from polyether ether ketone (PEEK). The insulating member 128 may help insulate the various components of the device 100 from the heat generated in the susceptor 132.

The insulating member 128 can also fully or partially support the first and second inductor coils 124, 126. For example, as shown in Figure 2, the first and second inductor coils 124, 126 are positioned around the insulating member 128 and are in contact with a radially outward surface of the insulating member 128. In some examples the insulating member 128 does not abut the first and second inductor coils 124, 126. For example, a small gap may be present between the outer surface of the insulating member 128 and the inner surface of the first and second inductor coils 124, 126.

In a specific example, the susceptor 132, the insulating member 128, and the first and second inductor coils 124, 126 are coaxial around a central longitudinal axis of the susceptor 132.

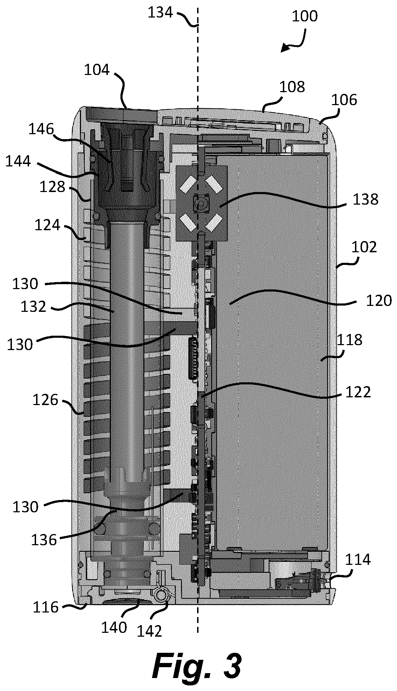

Figure 3 shows a side view of device 100 in partial cross-section. The outer cover 102 is present in this example. The rectangular cross-sectional shape of the first and second inductor coils 124, 126 is more clearly visible.

The device 100 further comprises a support 136 which engages one end of the susceptor 132 to hold the susceptor 132 in place. The support 136 is connected to the second end member 116.

The device may also comprise a second printed circuit board 138 associated within the input interface 112.

The device 100 further comprises a second lid/cap 140 and a spring 142, arranged towards the distal end of the device 100. The spring 142 allows the second lid 140 to be opened, to provide access to the susceptor 132. A user may open the second lid 140 to clean the susceptor 132 and/or the support 136.

The device 100 further comprises an expansion chamber 144 which extends away from a proximal end of the susceptor 132 towards the opening 104 of the device. Located at least partially within the expansion chamber 144 is a retention clip 146 to abut and hold the article 110 when received within the device 100. The expansion chamber 144 is connected to the end member 106.

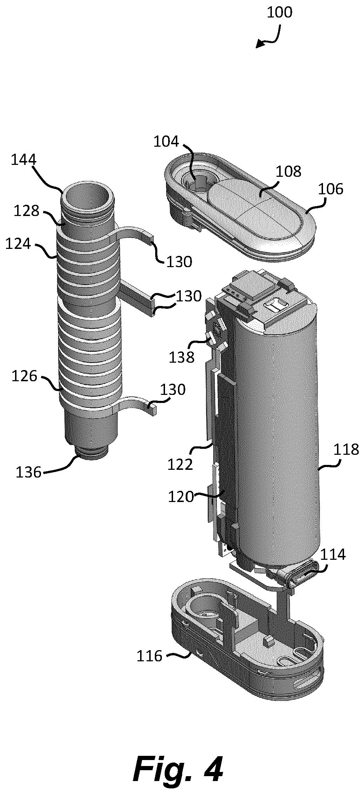

Figure 4 is an exploded view of the device 100 of Figure 1, with the outer cover 102 omitted.

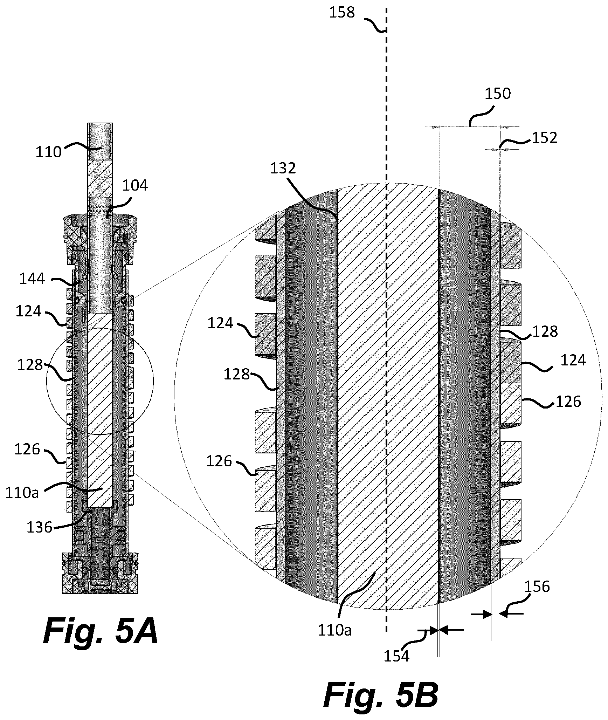

Figure 5A depicts a cross section of a portion of the device 100 of Figure 1. Figure 5B depicts a close-up of a region of Figure 5A. Figures 5A and 5B show the article 110 received within the susceptor 132, where the article 110 is dimensioned so that the outer surface of the article 110 abuts the inner surface of the susceptor 132. This ensures that the heating is most efficient. The article 110 of this example comprises aerosol generating material 110a. The aerosol generating material 110a is positioned within the susceptor 132. The article 110 may also comprise other components such as a filter, wrapping materials and/or a cooling structure.

Figure 5B shows that the outer surface of the susceptor 132 is spaced apart from the inner surface of the inductor coils 124, 126 by a distance 150, measured in a direction perpendicular to a longitudinal axis 158 of the susceptor 132. In one particular example, the distance 150 is about 3mm to 4mm, about 3mm to 3.5mm, or about 3.25mm.

Figure 5B further shows that the outer surface of the insulating member 128 is spaced apart from the inner surface of the inductor coils 124, 126 by a distance 152, measured in a direction perpendicular to a longitudinal axis 158 of the susceptor 132. In one particular example, the distance 152 is about 0.05mm. In another example, the distance 152 is substantially 0mm, such that the inductor coils 124, 126 abut and touch the insulating member 128.

In one example, the susceptor 132 has a wall thickness 154 of about 0.025mm to 1mm, or about 0.05mm.

In one example, the susceptor 132 has a length of about 40mm to 60mm, about 40mm to 45mm, or about 44.5mm.

In one example, the insulating member 128 has a wall thickness 156 of about 0.25mm to 2mm, about 0.25mm to 1mm, or about 0.5mm.

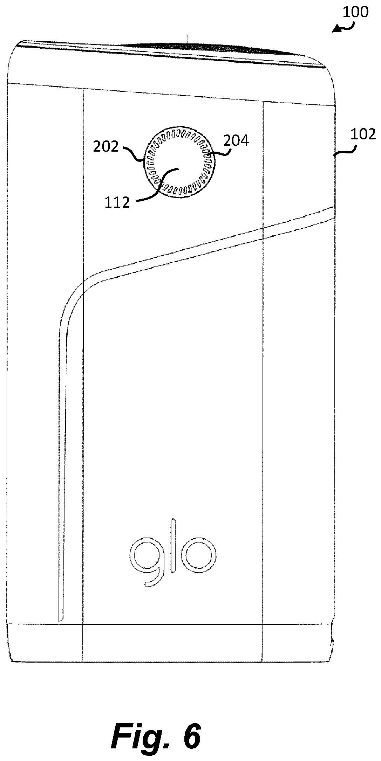

Figure 6 depicts a front view of the device 100. As briefly mentioned above, the device may comprise an input interface 112. In some examples the user may interact with the input interface 112 to operate the device 100. Arranged in proximity to the input interface 112 may be an indicator assembly, which can indicate the occurrence of one or more events to a user, such as when the device is ready for use and/or when the device has finished operating. The indicator assembly may also indicate a mode in which the device 100 is operating.

Figure 6 depicts an outer member 202 positioned above (i.e. in front of) an indicator assembly. In other examples, the indicator assembly may be positioned elsewhere on the device. In the examples described herein, the indicator assembly comprises a visual component configured to provide a visual indication. The visual component comprises a plurality of LEDs which emit electromagnetic radiation, such as light, to indicate certain events to a user. It will be appreciated that indicator assembly may additionally or alternatively comprise a haptic component or an audible indicator. In the present device 100, the indicator assembly comprises a visual component and a haptic component.

The outer member 202 forms the outermost component of the input interface 112. A user may press the outer member 202 to interact with the device 100. As will be described in more detail below, the outer member 202 comprises a plurality of apertures 204 through which light from a plurality of LEDs can pass.

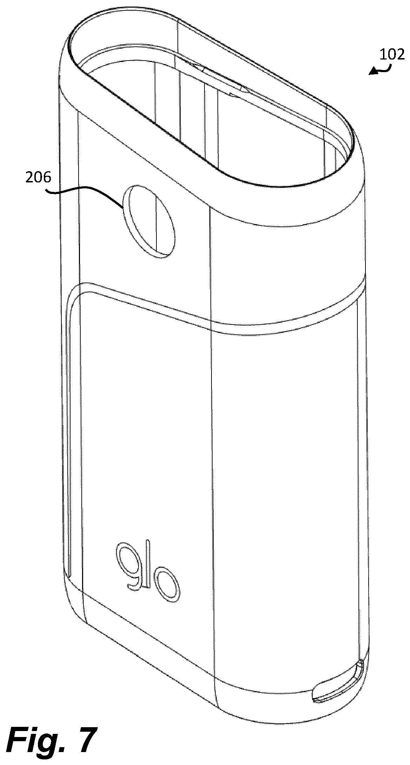

Figure 7 depicts the housing 102 (also known as the outer cover) of the device 100. The housing 102 delimits an opening 206. The outer member (not shown in Figure 7) can be arranged within the opening 206. For example, the outer member may be arranged flush with the outer surface of the housing 102, or may be raised above or below the outer surface of the housing 102.

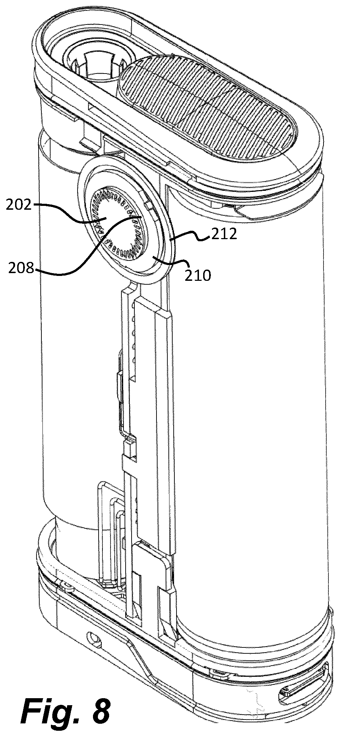

Figure 8 depicts the device 100 without the housing 102 in place. In this example, the outer member 202 is adhered to a light-shaping member 210 via an adhesive layer 208. The adhesive in the adhesive layer 208 may partially or fully cover an inner surface of the outer member 202. Extending around the light-shaping member 210 is a sealing member 212.

In some examples the outer member 202, the adhesive layer 208, the light-shaping member 210 and sealing member 212 may be omitted from the device.

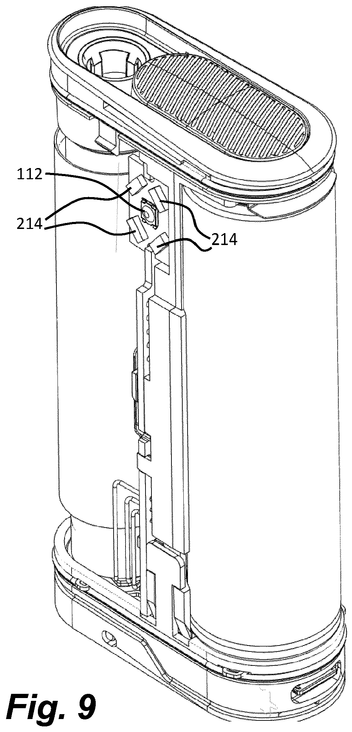

Figure 9 depicts the device 100 with the outer member 202, light-shaping member 210 and sealing member 212 removed. The device 100 comprises a visual component comprising four LEDs 214, although in other examples there may be other numbers of LEDs, such as one or more LEDs 214. The LEDs 214 are positioned below the outer member 202 such that light travels from the LEDs 214 through the plurality of apertures 204 formed in the outer member 202. The light therefore also passes through the light-shaping member 210 and the adhesive layer 208. There may also be one or more additional components arranged between the LEDs 214 and the outer member 202.

In the example of Figure 9, the LEDs 214 are arranged around the input interface 112 which is configured to detect interactions from a user. For example, a user may press or otherwise operate the outer member 202 which in turn is detected by the input interface 112. The input interface 112 may be button or switch which is operated when a force is applied by the user to the outer member 202. In another example the input interface 112 and the outer member 202 may be part of a capacitive sensor which detects when a user touches the outer member 202.

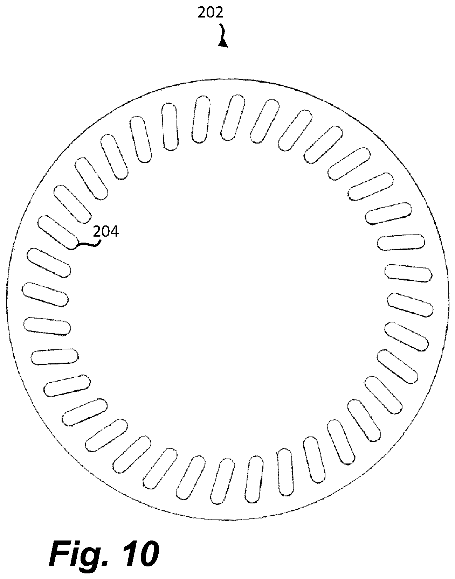

Figure 10 depicts a front view of the outer member 202. As mentioned, the outer member 202 defines a plurality of apertures 204. In this example, the apertures 204 each form slots with a length and a width.

Preferably, the apertures 204 are arranged towards the perimeter/periphery/outer circumference of the outer member 202. As shown in Figure 10, the apertures 204 are arranged closer to the periphery of the outer member 202 than the centre of the outer member 202. This can allow the apertures 204 to be exposed (and therefore light to be seen) even when the user is pressing the outer member 202. The user may be more likely to press/hold the centre of the outer member 202 rather than an edge of the outer member 202.

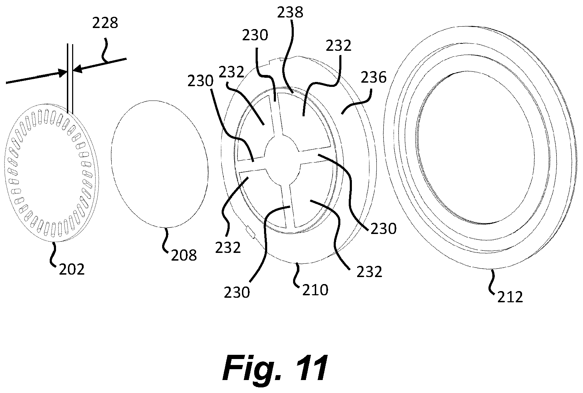

Figure 11 is an exploded diagram showing some of the components of the device 100. As mentioned, the device 100 may comprise an adhesive layer 208 arranged between the LEDs 214 and the outer member 202. In the example shown, the adhesive layer is the same shape and size as the outer member 202 such that the adhesive covers the apertures 204. Light can then pass through the adhesive layer 208 before passing through the apertures 204. The adhesive layer 208 can therefore be transparent or translucent. A translucent adhesive layer 208 can help diffuse the light from the LEDs such that "hot spots" are avoided. A hot spot is a region where the light has a higher intensity than surrounding regions.

In some examples, the outer member 202 is attached to a light-shaping member 210 via the adhesive layer 208. In the example shown, the light shaping-member 210 comprises one or more opaque regions 230 (which may be joined together) and one or more translucent or transparent regions 232 (which may also be joined together). The translucent or transparent regions 232 may be known as light-pipes, since they guide light through the light-shaping member 210. Light from the LEDs 214 can pass through the translucent or transparent regions 232 but is blocked by opaque regions 230. The opaque regions 230 therefore reduce the intensity of light passing through a subset of the apertures 204 (i.e. those arranged above the opaque regions 230). The opaque regions 230 and the translucent or transparent regions 232 may be regions of a single monolithic component, but one or both regions may have been treated to give the region its specific optical property. In another example, the opaque regions 230 and the translucent or transparent regions 232 are separate components which are overmolded.

In this example, the light-shaping member comprises an opaque region 238 extending around the periphery/perimeter/circumference of the light-shaping member 210. This can prevent light from leaking around the outside of the outer member 202. The opaque region may be an outer ring, for example.

In the present example, the device 100 comprises four LEDs 214, and each of the LEDs 214 is positioned between adjacent opaque regions 230 such that the light from the LEDs separates into 4 quadrants. In other words, the LEDs 214 may be arranged below the transparent or translucent regions. By separating the light into the different regions, different indications can be provided to a user. For example, the number of illuminated quadrants can specify certain events to a user. Accordingly, light may be blocked by the opaque regions such that the light may not pass through some of the apertures.

In some examples the regions between the opaque regions 230 are openings and therefore do not comprise translucent or transparent material.

Arranged between the light-shaping member 210 and the LEDs 214 is a sealing member 212, such as a gasket. The sealing member 212 has an outer diameter that is larger than the outer diameters of the outer member 202 and the light shaping member 210. In some examples the sealing member 210 abuts an inner surface of the housing 102 to stop liquid and dust from entering the device 100.

### Indicating that the device is ready for use

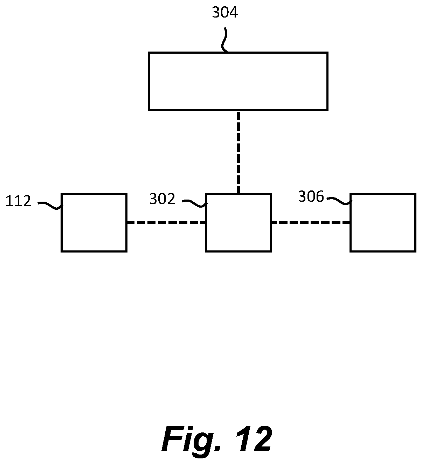

Figure 12 depicts a diagrammatic representation of a system comprising a controller 302 (such as one or more processors), a heater assembly 304, an indicator assembly 306 and the input interface 112. The controller 302 is communicatively coupled to the heater assembly 304, the indicator assembly 306 and the input interface 112 via one or more wired or wireless connections (shown as dashed lines).

The controller 302 may be located on the PCB 122, for example. The controller 302 can control operations of the device 100, such as causing the heater assembly 304 to heat aerosol generating material. In some examples, the controller 302 receives signals from the input interface 112, and responsively controls the heater assembly 304 and indicator assembly 306. A user can provide an input to the input interface 112 to operate the device. In certain examples a heating mode is selected via the input interface 112.

As mentioned above, the indicator assembly 306 can indicate the occurrence of one or more events to a user. To cause the indicator assembly 306 to provide an indication, the controller 302 can send a signal or instruction to the indicator assembly 306. In the examples of Figures 6-11, the indicator assembly 306 comprises a visual component comprising a plurality of LEDs 214. It will be appreciated that the following discussion can also be applied to other types of indicator assembly 306.

In the following examples, the heater assembly 304 comprises one or more inductor coils which generate one or more magnetic fields to heat a susceptor. The controller 302 can cause the inductor coil(s) of the device 100 to generate a varying magnetic field. For example, the controller 302 can send one or more signals to the inductor coil(s). Once the inductor coil(s) have begun generating the varying magnetic field, the susceptor 132 is heated, which in turn heats any aerosol generating material located near to the susceptor 132. It will be appreciated that the following description may also apply to other types of heater assembly 304.

The controller 302 may cause one or more inductor coils to heat the susceptor to between about 240°C and about 290°C. In a specific example, the device is configured to operate in one of a first mode and a second mode, where the first and second modes are heating modes. In one example, when the device is operating in a first (default) mode, the controller 302 may cause the first inductor coil 124 to heat a first region of the susceptor 132 to between about 240°C and about 260°C, such as about 250°C. In another example, the device may be operating in a second (boost) mode, and the controller 302 may cause the first inductor coil 124 to heat a first region of the susceptor 132 to between about 270°C and about 290°C, such as about 280°C.

The second inductor coil 126 may generate a second magnetic field at a later time during the heating session. For example, the second inductor coil 126 may generate the second magnetic field between about 60 seconds and about 130 seconds after the first inductor coil 124 generates a first magnetic field. The second inductor coil is arranged to heat a second region of the susceptor 132. In some examples, both inductor coils 124, 126 operate at the same time.

After the first inductor coil 124 begins heating the susceptor 132, the first region of the susceptor 132 may reach the desired temperature within 2 seconds. However, it may take longer for the heat to penetrate into the aerosol generating material. For example, it may take up to 60 seconds for the aerosol generating material to approach the temperature of the susceptor 132. Due to the efficient nature of inductive heating, the aerosol produced within the first 10-30 seconds may still be suitable for inhalation, despite the aerosol generating material not being fully heated.

Accordingly, the controller 302 may be configured to cause the indicator assembly 306 of the device to indicate that the device is ready for use within a predetermined period of time after causing the first inductor coil to generate the varying magnetic field. For example, the predetermined period of time may be less than about 30 seconds, less than about 20 seconds, less than about 15 seconds, or less than about 10 seconds after causing the inductor coil to generate the varying magnetic field. The controller 302 may initiate a timer at the moment it causes the inductor coil to generate the varying magnetic field (or shortly thereafter).

In a particular example, the predetermined period of time is dependent upon the mode in which the device is operating. For example, if the device is operating in the second, boost mode, the predetermined period of time is less than the predetermined period of time for when the device is operating in the first, default mode. This may be because the aerosol generating material is heated to a higher temperature in a shorter period of time in the second boost mode, which can mean that the device is ready for use sooner.

In one example, the LEDs 214 emit light to indicate when the device 100 is ready to use. For example, one or all of the LEDs 214 may be illuminated when the device 100 is ready for use (i.e. after the predetermined period of time has elapsed).

In a specific example, the number of LEDs 214 which are illuminated indicates when the device is ready for use. For example, when all of the LEDs 214 are illuminated, the device may be ready for use.

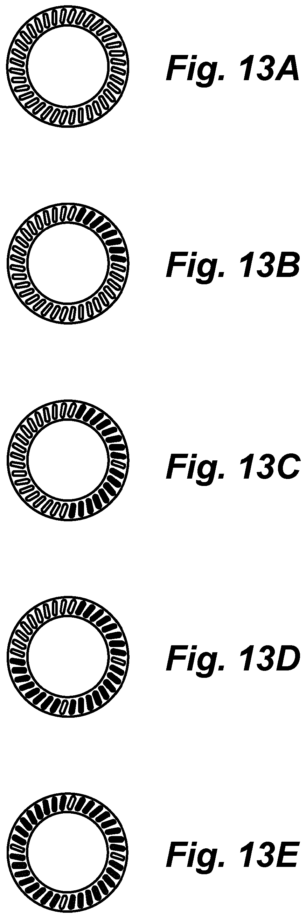

Figure 13A depicts the outer member 202 positioned above the four LEDs 214. No light passes through the apertures 204 because none of the LEDs 214 are illuminated. At this moment in time, the user may not yet have pressed the input interface 112, so the controller 302 has not yet received an input from the input interface 112, nor has the controller 302 caused the inductor coil 124 to generate the varying magnetic field. When an input has been detected, the controller 302 causes the inductor coil 124 to generate the varying magnetic field. Figure 13A also shows the outer member 202 at a moment in time after the user has pressed the input interface 112, but before any of the LEDs 214 have been switched on.

Figure 13B depicts the outer member 202 a first threshold period of time after the controller 302 caused the inductor coil 124 to generate the varying magnetic field. The first threshold period may be 5 seconds, for example. At this time, one of the LEDs has been illuminated, and light passes through a subset of the apertures 204 to illuminate one quadrant of the outer member 202.

Figure 13C depicts the outer member 202 a second threshold period of time after the controller 302 caused the inductor coil 124 to generate the varying magnetic field. The second threshold period may be 10 seconds, for example. At this time, two of the LEDs have been illuminated, and light passes through a subset of the apertures 204 to illuminate two quadrants of the outer member 202.

Figure 13D depicts the outer member 202 a third threshold period of time after the controller 302 caused the inductor coil 124 to generate the varying magnetic field. The third threshold period may be 15 seconds, for example. At this time, three of the LEDs have been illuminated, and light passes through a subset of the apertures 204 to illuminate three quadrants of the outer member 202.

Figure 13E depicts the outer member 202 a fourth threshold period of time after the controller 302 caused the inductor coil 124 to generate the varying magnetic field. The fourth threshold period may be 20 seconds, for example. At this time, all four of the LEDs have been illuminated, and light passes through the majority of the apertures 204 to illuminate four quadrants of the outer member 202. Accordingly, when all four LEDs are illuminated, the indicator assembly 306 indicates that the device is ready for use. This occurs within 20 seconds of causing the inductor coil 124 to generate the magnetic field.

In another example, the first threshold period of time may be between about 3 seconds and 5 seconds, the second threshold period of time may be between about 6 seconds and 10 seconds, the third threshold period of time may be between about 9 seconds and 15 seconds and the fourth threshold period of time may be between about 12 seconds and 20 seconds. The first, second, third and fourth threshold periods of time may be dependent upon the mode in which the device is operating. For example, if the device is operating in the first default mode, the first, second, third and fourth threshold periods may be longer than the respective first, second, third and fourth threshold periods for when the device is operating in the second, boost mode. This can be because the aerosol generating material heats up quicker in the second, boost mode.

In a particular example, the indicator assembly 306 may further comprise a haptic component, where the haptic component is configured to provide haptic feedback to indicate that the device has begun heating the aerosol generating material. This can be useful if the first LED is not illuminated at the time the inductor coil begins to generate the magnetic field, but is instead illuminated after the first threshold period. The haptic feedback may be indicative of the mode in which the device is operating.

In some examples the first LED may be illuminated at substantially the same time the controller 302 causes the inductor coil 124 to generate the magnetic field (i.e. rather than after a first threshold period of time has elapsed). Accordingly, the visual component of the indicator assembly 306 may also indicate that the device has begun heating the aerosol generating material. A haptic component may also provide a substantially simultaneous indication at the time the inductor coil begins to generate the magnetic field.

In another example, the indicator assembly 306 may comprise a haptic component, where the haptic component is configured to provide haptic feedback to indicate that the device is ready for use. This may occur instead of, or in addition to any other types of indications. For example, the indicator assembly 306 may provide both a visual indication and haptic feedback to indicate that the device is ready for use.

In another example, the indicator assembly 306 may comprise an audible indicator, where the audible indicator is configured to emit sound to indicate that the device is ready for use. This may occur instead of, or in addition to any other types of indications. For example, the indicator assembly 306 may provide both a visual indication and emit sound to indicate that the device is ready for use.

### Input Interface

As mentioned above, the controller 302 detects an input from the input interface 112 and responsively causes the inductor coil 124 to generate the varying magnetic field. In the present example, the input interface 112 comprises a single button and the input interface 112 sends a signal to the controller to indicate that the user has operated the input interface. In a specific example, the signal indicates that the user has released the button. A user can therefore press and hold the button, and the controller 302 causes the inductor coil 124 to generate the varying magnetic field when the button has been released.

In a specific example, the user can press and hold the button for different lengths of time, and the device is operated in a particular mode depending upon the length of time. The input received from the input interface 112 may therefore also comprise a signal indicating the length of time that the button was pressed, for example as an indication of the time itself or by sending two signals, one for press and one for release, which can be timed by the controller 302. The controller 302 may be configured to cause the inductor coil 124 to generate the varying magnetic field in response to receiving the signal indicating that the button has been released and in response to determining that the length of time that the button has been pressed is greater than or equal to a threshold time period. If the length of time is less than the threshold time period, the device 100 does not begin heating. In a particular example, if the length of time is less than the threshold time period, the device 100 may display a power level of device's power source 118.

As mentioned, the device 100 may be configured to operate in a first mode or a second mode. Thus, in a particular example, if the length of time that the button has been pressed is greater than or equal to a first threshold time period and is less than a second threshold time period, the controller 302 is configured to operate the device in the first mode. If the length of time that the button has been pressed is greater than or equal to the second threshold time period, the device is configured to operate in the second mode. The first threshold time period may be 3 seconds, and the second threshold time period may be 5 seconds, for example. Thus, using a single button the user can select different modes. If the user holds down the button for longer than 3 seconds, but less than 5 seconds, the device operates in the first mode.

In a particular example, if the length of time that the button has been pressed is greater than or equal to a third threshold time period, the device is configured to operate in a settings mode. A settings mode can allow the user to configure settings of the device. The third threshold time period may be greater than the second threshold time period. In a particular example, the third threshold time period is 8 seconds. If the user holds down the button for longer than 5 seconds, but less than 8 seconds, the device operates in the second mode.

In another example, if the length of time that the button has been pressed is greater than or equal to a fourth threshold time period, but less than first time period, the device is configured to display a power level of the power source 118. The fourth threshold time period may be 1 second, for example. If the user holds down the button for longer than 1 second and less than 3 seconds, the device can display the power level. The power level may be indicated by the indicator assembly 306. For example, if the power level is between 0% and 25%, one of the four LEDs 214 may be illuminated. If the power level is between 25% and 50%, two of the LEDs 214 may be illuminated. If the power level is between 50% and 75%, three of the LEDs 214 may be illuminated. If the power level is between 75% and 100%, four of the LEDs 214 may be illuminated.

The above describes just one specific type of input interface 112. In another example the user selects the operating mode using a touchscreen. In another example, there may be one or more input interfaces. For example, to operate the device in a first mode the user may operate a first input interface and to operate the device in a second mode the user may operate a second input interface. The controller 302 may therefore be configured to cause the inductor coil to generate the varying magnetic field in response to an input received from one of the first and second input interfaces.

### Indicating that the device has finished operating

As described above, the indicator assembly 306 can indicate that the device is ready for use, or to indicate that the device has begun heating the aerosol generating material. Alternatively, or additionally, the indicator assembly 306 can indicate that the device has finished operating or is about to finish operating. In certain examples, the indicator assembly 306 is configured to indicate the time left until the device finishes operating.

The device may be configured to heat the aerosol generating material for a predetermined period of time. The controller 302 may therefore cause the indicator assembly 306 to indicate that the device has finished operating or is about to finish operating within a predetermined period of time after causing the inductor coil to generate the varying magnetic field. The predetermined period of time may be about three minutes, three minutes and thirty seconds, or four minutes for example. In some examples the predetermined time depends upon the mode in which the device is operating.

In another example, the device may be configured to heat the aerosol generating material for a predetermined number of "puffs". For example, the device may comprise a puff sensor to determine when a user draws or puffs on the device. The controller 302 may therefore cause the indicator assembly 306 to indicate that the device has finished operating or is about to finish operating when a predetermined number of puffs have been detected by the puff sensor.

In another example, the device may be configured to heat the aerosol generating material until a predetermined volume of air has been drawn through the device. For example, the device may comprise a flow sensor to determine volume of air flowing through the device over a heating session. The controller 302 may therefore cause the indicator assembly 306 to indicate that the device has finished operating or is about to finish operating when a predetermined volume of air has flowed through the device.

In another example, the device may be configured to heat the aerosol generating material until a predetermined amount of energy has been used by the device. For example, the device may comprise an energy sensing module to determine the amount of energy used by the device during a heating session. The controller 302 may therefore cause the indicator assembly 306 to indicate that the device has finished operating or is about to finish operating when a predetermined amount of energy has been used by the device.

In another example, the device may be configured to heat the aerosol generating material and may cause the device to stop heating when a battery level decreases below a threshold. For example, the device may comprise an energy sensing module to determine the amount of energy remaining in the battery. The controller 302 may therefore cause the indicator assembly 306 to indicate that the device has finished operating or is about to finish operating when the remaining energy falls below a threshold.

In one example, the indicator assembly 306 indicates that the device has finished operating or is about to finish operating by ceasing to provide any indications. For example, while the device is operating, a visual component, such as one or more LEDs, may visually indicate that the device is operating. When the visual indication stops, the user may be informed that the device has finished operating. For example, if the one or more LEDs are illuminated while the device is operating, they may be switched off when the device has finished operating, thus providing an indication to the user.

The indicator assembly 306 indicates that the device has finished operating by providing a particular indication. A visual component provides a particular indication to indicate that the device has finished operating or is about to finish operating. The visual indication is different to a previous visual indication. For example, if one or more LEDs are illuminated while the device is operating, they may flash in a particular pattern to indicate that the device has finished operating or is about to finish operating.

In a particular example, the indicator assembly 306 may comprise a haptic component, where the haptic component is configured to provide haptic feedback to indicate that the device has finished operating or is about to finish operating. In another example, the indicator assembly 306 may comprise an audible indicator, where the audible indicator is configured to emit sound to indicate that the device has finished operating or is about to finish operating. Two or more different types of indication may be provided.

In some examples, the indicator assembly 306 is configured to indicate the time left until the device finishes operating. For example, an indication may be provided at various points in time as the device approaches its finishing time.

In one example, a haptic component may provide haptic feedback 20 seconds from the end of the heating session, and may also provide haptic feedback 15 seconds from the end of the heating session, 10 seconds from the end of the heating session, 5 seconds from the end of the heating session and at the end of the heating session. The haptic feedback provided at each moment in time may be the same or different. For example, the feedback may become more intense or may last longer towards the end of the heating session.

In another example, the indicator assembly 306 comprises a plurality of LEDs, and the number of illuminated LEDs indicates the time left until the device finishes operating. For example, when the device is operating there may be a first number of LEDs illuminated and when the device has finished operating there may be a second number of LEDs illuminated, where the second number is less than the first number. The second number may be zero, for example. The first number may be all of the LEDs. The LEDs may therefore "count down" as the device gets closer to finishing.

In a particular example there are a plurality of LEDs, such as four LEDs, and the LEDs are sequentially switched off as the end of the heating session approaches. Figure 13E may depict the outer member 202 as the device is operating. The first and/or second inductor coils may or may not be active at this time. At this time, all four LEDs are illuminated to indicate that the user can still use the device. There may be a threshold period of time remaining until the device finishes operating. For example, there may be 20 seconds remaining until the device finishes operating.

In one example the device is said to have "finished operating" at the time the first and/or second inductor coil has ceased generating the varying magnetic field. In another example, the device is said to have "finished operating" at the time the aerosol temperature/volume is considered to fall below an acceptable level, which may be after the point at which the first and/or second inductor coil has ceased generating the varying magnetic field.

Figure 13D may depict the outer member 202 at a later time than that shown in Figure 13E. For example, there may only be 15 seconds remaining until the device finishes operating. At this time, one of the four LEDs has been switched off and light passes through a subset of the apertures 204 to illuminate three quadrants of the outer member 202.

Figure 13C may depict the outer member 202 at a later time than that shown in Figure 13D. For example, there may only be 10 seconds remaining until the device finishes operating. At this time, two of the four LEDs have been switched off and light passes through a subset of the apertures 204 to illuminate two quadrants of the outer member 202.

Figure 13B may depict the outer member 202 at a later time than that shown in Figure 13C. For example, there may only be 5 seconds remaining until the device finishes operating. At this time, three of the four LEDs have been switched off and light passes through a subset of the apertures 204 to illuminate one quadrant of the outer member 202.

Figure 13A may depict the outer member 202 at a later time than that shown in Figure 13B. For example, the device may have finished operating. At this time, all four LEDs have been switched off and no light is visible. The indicator assembly 306 therefore indicates that the device has finished operating, while also indicating the time left until the device has finished operating.

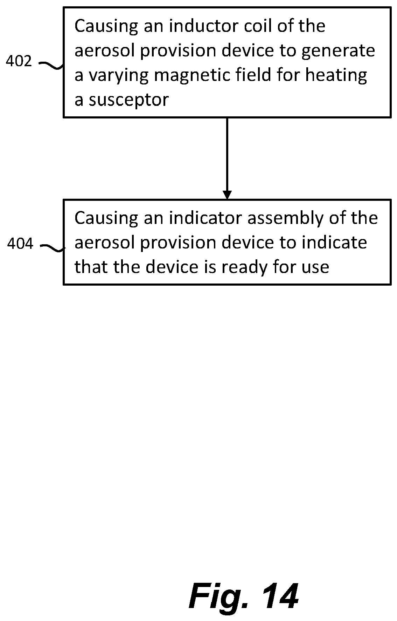

Figure 14 is a flow diagram of a method of operating an aerosol provision device. The method comprises, at block 402, causing an inductor coil of an aerosol provision device to generate a varying magnetic field for heating a susceptor. The method comprises, at block 404, causing an indicator assembly of the aerosol provision device to indicate that the device is ready for use within a predetermined period of time after causing the inductor coil to generate the varying magnetic field, wherein the predetermined period of time is less than or equal to about 20 seconds. In one example, the indicator assembly indicates that the device is ready for use at a predetermined period of time after causing the inductor coil to generate the varying magnetic field.

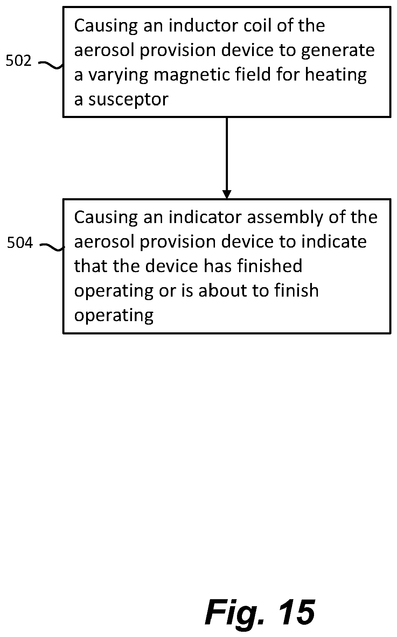

Figure 15 is a flow diagram of another method of operating an aerosol provision device. The method comprises, at block 502, causing an inductor coil of the aerosol provision device to generate a varying magnetic field for heating a susceptor. The method comprises, at block 504, causing an indicator assembly of the aerosol provision device to indicate that the device has finished operating or is about to finish operating within a predetermined period of time after causing the inductor coil assembly to begin heating the aerosol generating material.

The above embodiments are to be understood as illustrative examples of the invention. Further embodiments of the invention are envisaged. It is to be understood that any feature described in relation to any one embodiment may be used alone, or in combination with other features described, and may also be used in combination with one or more features of any other of the embodiments, or any combination of any other of the embodiments. Furthermore, equivalents and modifications not described above may also be employed without departing from the scope of the invention, which is defined in the accompanying claims.

## Claims

1. An aerosol provision device (100), comprising:
an inductor coil (124, 126);
an opening (104) in one end of the device through which an article (110) comprising aerosol generating material may be inserted;
a heating zone configured to receive the article, the article comprising aerosol generating material and a susceptor (132) arranged to heat the aerosol generating material, wherein the susceptor is heatable by the inductor coil, wherein the article is removable from the heating zone after use; and wherein the inductor coil is helical and encircles at least part of the heating zone;
an indicator assembly (306); and
a controller (302), configured to cause the coil to begin heating the susceptor;
and
the aerosol provision device being **characterised in that** the controller is configured to cause the indicator assembly to indicate that the device has finished operating or is about to finish operating within a predetermined period of time after causing the coil to begin heating the aerosol generating material;
wherein the device further comprises an input interface (112), and the controller is configured to cause the coil to heat the susceptor in response to an input received from the input interface;
wherein the input interface is a button;
wherein the indicator assembly comprises a visual component comprising one or more LEDs (214) configured to provide a visual indication that the device is ready for use;
wherein the indicator assembly comprises a haptic component configured to provide haptic feedback to indicate that the device is ready for use;
wherein the indicator assembly is configured to provide different haptic feedback patterns to indicate the time left until the device finishes operating at different times;
wherein the indicator assembly is configured to cause the one or more LEDs of the visual component to provide a particular indication to indicate that the device is about to finish operating which is different to a previous visual indication.

2. An aerosol provision device (100) of claim 1, wherein the controller (302) is configured to cause the indicator assembly (306) to indicate that the device is ready for use within a predetermined period of time after causing the coil (124, 126) to heat the susceptor (132) or after a predetermined temperature has been reached in the device, wherein the predetermined period of time is less than about 30 seconds.

3. An aerosol provision device (100) according to claim 2, wherein the predetermined period of time is less than about 15 seconds after causing the coil (124, 126) to heat the susceptor (132).

4. An aerosol provision device (100) according to any of claims 1 to 3, wherein the device is configured to operate in one of a first mode and a second mode, and when the device is operated in the second mode the coil is configured to cause the aerosol generating material to be heated to a higher temperature than when the device is operated in the first mode, and wherein the predetermined period of time is a first predetermined time when the device is operating in the first mode and a second predetermined time when the device is operating in the second mode, the second predetermined time being different than the first predetermined time.

5. An aerosol provision device (100) according to any of claims 1 to 4, wherein the device is configured to operate in one of a first mode and a second mode, and when the device is operated in the second mode the coil is configured to cause the susceptor (132) to be heated to a higher temperature than when the device is operated in the first mode, and wherein the predetermined period of time is a first predetermined time when the device is operating in the first mode and a second predetermined time when the device is operating in the second mode, the second predetermined time being less than the first predetermined time.

6. An aerosol provision device (100) according to any of claims 1 to 5, wherein the input comprises a signal indicating the button has been released.

7. An aerosol provision device (100) according to claim 6, wherein the input further comprises a signal indicating a length of time that the button has been pressed and the controller (302) is configured to cause the coil (124, 126) to heat the susceptor (132) in response to:
receiving the signal indicating that the button has been released; and
determining that the length of time that the button has been pressed is greater than or equal to a threshold time period.

8. An aerosol provision device (100) according to any of claims 1 to 7, wherein the device further comprises a puff detector for detecting when a user takes a puff on the device, and the controller (302) is configured to cause the coil to heat the susceptor (132) in response to the puff detector detecting when the user takes a puff on the device.

9. An aerosol provision device (100) according to any of claims 1 to 8, wherein the indicator assembly (306) comprises an audible indicator configured to emit sound to indicate that the device is ready for use.

10. An aerosol provision device (100) according to any of claims 1 to 9, wherein the coil is a first coil (124), and the device further comprises a second coil (126) for heating the susceptor (132), and wherein:
the first coil is adjacent the second coil in a direction along a longitudinal axis of the device;
the controller (302) is configured to cause the second coil to heat the susceptor after causing the indicator assembly (306) to indicate that the device is ready for use; and
in use, the aerosol is drawn along a flow path of the device towards a proximal end of the device, and the first coil is arranged closer to the proximal end of the device than the second coil.

11. An aerosol provision device (100) according to any of claims 1 to 10, wherein the indicator assembly (306) is configured to provide a haptic feedback indication that the inductor coil (124, 126) has begun to generate the varying magnetic field.

12. An aerosol provision device (100) according to any of claims 1 to 11, wherein each of the plurality of LEDs (214) are configured to be switched off when the device has finished operating.

13. A system, comprising:
the device (100) of any of claims 1 to 12, further comprising the article (110), wherein the susceptor is configured to generate heat in the presence of a varying magnetic field.

14. A method of operating an aerosol provision device (100),
comprising:
causing an inductor coil (124, 126) of the aerosol provision device to begin heating a susceptor (132) heatable by the inductor coil to heat aerosol generating material of an article (110) received by a heating zone and comprising the susceptor and aerosol generating material, the article being inserted through an opening (104) in one end of the device, and the article being removable from the heating zone after use; wherein the inductor coil is helical and encircles at least part of the heating zone;
causing an indicator assembly (306) of the aerosol provision device to indicate that the device has finished operating or is about to finish operating within a predetermined period of time after causing the coil to begin heating the aerosol generating material;
causing the coil to heat the susceptor in response to an input received from an input interface (112); wherein the input interface is a button;
providing, by a visual component of the indicator assembly comprising one or more LEDs (214), a visual indication that the device is ready for use;
providing, by a haptic component of the indicator assembly, haptic feedback to indicate that the device is ready for use;
providing, by the indicator assembly, different haptic feedback patterns to indicate the time left until the device finishes operating at different times; and
causing the one or more LEDs of the visual component of the indicator assembly to provide a particular indication to indicate that the device is about to finish operating which is different to a previous visual indication.

15. A method according to claim 14, comprising:
causing an indicator assembly (306) of the aerosol provision device (100) to indicate that the device is ready for use within a predetermined period of time after causing the coil (124, 126) to heat the susceptor or after a predetermined temperature has been reached in the device, wherein the predetermined period of time is less than about 30 seconds.

## Patentansprüche

1. Aerosolbereitstellungsvorrichtung (100), umfassend:
eine Induktionsspule (124, 126);
eine Öffnung (104) in einem Ende der Vorrichtung, durch die ein Artikel (110), der ein Aerosol erzeugendes Material umfasst, eingeführt werden kann;
eine Heizzone, die zum Aufnehmen des Artikels ausgebildet ist, wobei der Artikel ein Aerosol erzeugendes Material und einen Suszeptor (132) umfasst, der zum Erhitzen des Aerosol erzeugenden Materials angeordnet ist, wobei der Suszeptor durch die Induktionsspule erhitzbar ist, wobei der Artikel nach Gebrauch aus der Heizzone entfernbar ist; und wobei die Induktionsspule spiralförmig ist und mindestens einen Teil der Heizzone umgibt;
eine Anzeigeanordnung (306); und
eine Steuereinheit (302), die so ausgebildet ist, dass sie die Spule veranlasst, damit zu beginnen, den Suszeptor zu erhitzen; und
wobei die Aerosolbereitstellungsvorrichtung **dadurch gekennzeichnet ist, dass** die Steuereinheit so ausgebildet ist, dass sie die Anzeigeanordnung dazu veranlasst, anzuzeigen, dass die Vorrichtung ihren Betrieb beendet hat oder kurz davor steht, ihren Betrieb innerhalb einer vorbestimmten Zeitspanne zu beenden, nachdem die Spule dazu veranlasst wurde, mit einem Erhitzen des Aerosol erzeugenden Materials zu beginnen;
wobei die Vorrichtung weiter eine Eingabeschnittstelle (112) umfasst und die Steuerung so ausgebildet ist, dass sie die Spule veranlasst, den Suszeptor als Reaktion auf eine von der Eingabeschnittstelle empfangene Eingabe zu erhitzen;
wobei die Eingabeschnittstelle eine Taste ist;
wobei die Anzeigeanordnung eine visuelle Komponente umfasst, die eine oder mehrere LEDs (214) umfasst, die so ausgebildet sind, dass sie eine visuelle Anzeige bereitstellen, dass die Vorrichtung betriebsbereit ist;
wobei die Anzeigeanordnung eine haptische Komponente umfasst, die so ausgebildet ist, dass sie eine haptische Rückmeldung bereitstellt, um anzuzeigen, dass die Vorrichtung betriebsbereit ist;
wobei die Anzeigeanordnung so ausgebildet ist, dass sie unterschiedliche haptische Rückmeldemuster bereitstellt, um zu unterschiedlichen Zeitpunkten die verbleibende Zeit bis zum Ende des Betriebs der Vorrichtung anzuzeigen;
wobei die Anzeigeanordnung so ausgebildet ist, dass sie die eine oder mehreren LEDs der visuellen Komponente veranlasst, eine bestimmte Anzeige bereitzustellen, um anzuzeigen, dass die Vorrichtung kurz vor dem Beenden ihres Betriebs steht, was sich von einer vorherigen visuellen Anzeige unterscheidet.

2. Aerosolbereitstellungsvorrichtung (100) nach Anspruch 1, wobei die Steuereinheit (302) so ausgebildet ist, dass sie die Anzeigeanordnung (306) veranlasst, anzuzeigen, dass die Vorrichtung innerhalb einer vorbestimmten Zeitspanne betriebsbereit ist, nachdem die Spule (124, 126) veranlasst wurde, den Suszeptor (132) zu erhitzen oder nachdem in der Vorrichtung eine vorbestimmte Temperatur erreicht wurde, wobei die vorbestimmte Zeitspanne weniger als etwa 30 Sekunden beträgt.

3. Aerosolbereitstellungsvorrichtung (100) nach Anspruch 2, wobei die vorbestimmte Zeitspanne weniger als etwa 15 Sekunden beträgt, nachdem die Spule (124, 126) veranlasst wurde, den Suszeptor (132) zu erhitzen.

4. Aerosolbereitstellungsvorrichtung (100) nach einem der Ansprüche 1 bis 3, wobei die Vorrichtung so ausgebildet ist, dass sie in einem von einem ersten Modus und einem zweiten Modus arbeitet, und wenn die Vorrichtung in dem zweiten Modus betrieben wird, die Spule so ausgebildet ist, dass sie veranlasst, dass das Aerosol erzeugende Material auf eine höhere Temperatur erhitzt wird als bei Betrieb der Vorrichtung in dem ersten Modus, wobei die vorbestimmte Zeitspanne eine erste vorbestimmte Zeit ist, wenn die Vorrichtung in dem ersten Modus arbeitet, und eine zweite vorbestimmte Zeit, wenn die Vorrichtung in dem zweiten Modus arbeitet, wobei die zweite vorbestimmte Zeit sich von der ersten vorbestimmten Zeit unterscheidet.

5. Aerosolbereitstellungsvorrichtung (100) nach einem der Ansprüche 1 bis 4, wobei die Vorrichtung so ausgebildet ist, dass sie in einem von einem ersten Modus und einem zweiten Modus arbeitet, und bei Betrieb der Vorrichtung in dem zweiten Modus, die Spule so ausgebildet ist, dass sie veranlasst, dass der Suszeptor (132) auf eine höhere Temperatur erhitzt wird als bei Betrieb der Vorrichtung in dem ersten Modus, und wobei die vorbestimmte Zeitspanne eine erste vorbestimmte Zeitspanne ist, wenn die Vorrichtung in dem ersten Modus arbeitet, und eine zweite vorbestimmte Zeit, wenn die Vorrichtung in dem zweiten Modus arbeitet, wobei die zweite vorbestimmte Zeit kürzer ist als die erste vorbestimmte Zeit.

6. Aerosolbereitstellungsvorrichtung (100) nach einem der Ansprüche 1 bis 5, wobei die Eingabe ein Signal umfasst, das anzeigt, dass die Taste losgelassen wurde.

7. Aerosolbereitstellungsvorrichtung (100) nach Anspruch 6, wobei die Eingabe weiter ein Signal umfasst, das eine Länge der Zeit angibt, während der die Taste gedrückt wurde, und die Steuereinheit (302) so ausgebildet ist, dass sie die Spule (124, 126) veranlasst, den Suszeptor (132) als Reaktion auf Folgendes zu erhitzen:
Empfangen des Signals, das anzeigt, dass die Taste losgelassen wurde; und
Feststellen, dass die Länge der Zeit, während der die Taste gedrückt wurde, größer oder gleich einer Schwellenzeitdauer ist.

8. Aerosolbereitstellungsvorrichtung (100) nach einem der Ansprüche 1 bis 7, wobei die Vorrichtung weiter einen Zugdetektor umfasst, um zu detektieren, wenn ein Benutzer einen Zug an der Vorrichtung nimmt, und die Steuereinheit (302) so ausgebildet ist, dass sie die Spule veranlasst, den Suszeptor (132) als Reaktion darauf zu erhitzen, dass der Zugdetektor detektiert, dass der Benutzer einen Zug an der Vorrichtung nimmt.

9. Aerosolbereitstellungsvorrichtung (100) nach einem der Ansprüche 1 bis 8, wobei die Anzeigeanordnung (306) eine akustische Anzeige umfasst, die so ausgebildet ist, dass sie einen Ton abgibt, um anzuzeigen, dass die Vorrichtung betriebsbereit ist.

10. Aerosolbereitstellungsvorrichtung (100) nach einem der Ansprüche 1 bis 9, wobei die Spule eine erste Spule (124) ist und die Vorrichtung weiter eine zweite Spule (126) zum Erhitzen des Suszeptors (132) umfasst, und wobei:
die erste Spule benachbart zu der zweiten Spule in einer Richtung entlang einer Längsachse der Vorrichtung angeordnet ist;
die Steuereinheit (302) so ausgebildet ist, dass sie die zweite Spule veranlasst, den Suszeptor zu erhitzen, nachdem sie die Anzeigeanordnung (306) veranlasst hat, anzuzeigen, dass die Vorrichtung betriebsbereit ist; und
bei einer Verwendung das Aerosol entlang eines Strömungswegs der Vorrichtung zu einem proximalen Ende der Vorrichtung gezogen wird, und wobei die erste Spule näher am proximalen Ende der Vorrichtung angeordnet ist als die zweite Spule.

11. Aerosolbereitstellungsvorrichtung (100) nach einem der Ansprüche 1 bis 10, wobei die Anzeigeanordnung (306) so ausgebildet ist, dass sie eine haptische Rückmeldung bereitstellt, die anzeigt, dass die Induktionsspule (124, 126) begonnen hat, das variierende Magnetfeld zu erzeugen.

12. Aerosolbereitstellungsvorrichtung (100) nach einem der Ansprüche 1 bis 11, wobei jede der Vielzahl von LEDs (214) so ausgebildet ist, dass sie ausgeschaltet wird, wenn die Vorrichtung ihren Betrieb beendet hat.

13. System, umfassend:
die Vorrichtung (100) nach einem der Ansprüche 1 bis 12, weiter umfassend den Artikel (110), wobei der Suszeptor so ausgebildet ist, dass er in der Gegenwart eines variierenden Magnetfelds Hitze erzeugt.

14. Verfahren zum Betreiben einer Aerosolbereitstellungsvorrichtung (100), umfassend:
Veranlassen einer Induktionsspule (124, 126) der Aerosolbereitstellungsvorrichtung damit zu beginnen, einen durch die Induktionsspule erhitzbaren Suszeptor (132) zu erhitzen, um Aerosol erzeugendes Material eines Artikels (110) zu erhitzen, der von einer Heizzone aufgenommen wird und den Suszeptor und das Aerosol erzeugende Material umfasst, wobei der Artikel durch eine Öffnung (104) in einem Ende der Vorrichtung eingeführt wird, und wobei der Artikel nach Gebrauch aus der Heizzone entfernbar ist; wobei die Induktionsspule spiralförmig ist und mindestens einen Teil der Heizzone umgibt;
Veranlassen einer Anzeigeanordnung (306) der Aerosolbereitstellungsvorrichtung zum Anzeigen, dass die Vorrichtung ihren Betrieb beendet hat oder kurz davor steht, ihren Betrieb innerhalb einer vorbestimmten Zeitspanne zu beenden, nachdem die Spule veranlasst wurde, mit einem Erhitzen des Aerosol erzeugenden Materials zu beginnen;
Veranlassen der Spule, den Suszeptor als Reaktion auf eine von einer Eingabeschnittstelle (112) empfangene Eingabe zu erhitzen; wobei die Eingabeschnittstelle eine Taste ist;
Bereitstellen einer visuellen Anzeige, dass die Vorrichtung betriebsbereit ist, durch eine visuelle Komponente der Anzeigeanordnung, die eine oder mehrere LEDs (214) umfasst;
Bereitstellen einer haptischen Rückmeldung durch eine haptische Komponente der Anzeigeanordnung, um anzuzeigen, dass die Vorrichtung betriebsbereit ist;
Bereitstellen verschiedener haptischer Rückmeldungsmuster durch die Anzeigeanordnung, um die verbleibende Zeit bis zum Ende des Betriebs der Vorrichtung zu unterschiedlichen Zeitpunkten anzuzeigen; und
Veranlassen der einen oder mehrere LEDs der visuellen Komponente der Anzeigeanordnung eine bestimmte Anzeige bereitzustellen, um anzuzeigen, dass die Vorrichtung kurz vor dem Beenden ihres Betriebs steht, die sich von einer vorherigen visuellen Anzeige unterscheidet.

15. Verfahren nach Anspruch 14, umfassend:
Veranlassen einer Anzeigeanordnung (306) der Aerosolbereitstellungsvorrichtung (100) anzuzeigen, dass die Vorrichtung innerhalb einer vorbestimmten Zeitspanne betriebsbereit ist, nachdem die Spule (124, 126) veranlasst wurde, den Suszeptor zu erhitzen oder nach Erreichen einer vorbestimmten Temperatur in der Vorrichtung, wobei die vorbestimmte Zeitspanne weniger als etwa 30 Sekunden beträgt.

## Revendications

1. Dispositif (100) de fourniture d'aérosol comprenant :
une bobine d'induction (124, 126) ;
une ouverture (104) dans une extrémité du dispositif à travers laquelle peut être inséré un article (110) comprenant une matière génératrice d'aérosol ;
une zone de chauffage configurée pour recevoir l'article, l'article comprenant la matière génératrice d'aérosol et un suscepteur (132) agencé pour chauffer la matière génératrice d'aérosol, dans lequel le suscepteur peut être chauffé par la bobine d'induction, dans lequel l'article peut être retiré de la zone de chauffage après utilisation ; et dans lequel la bobine d'induction est hélicoïdale et entoure au moins une partie de la zone de chauffage ;
un ensemble indicateur (306) ; et
un dispositif de commande (302), configuré pour amener la bobine à commencer à chauffer le suscepteur ; et
le dispositif de fourniture d'aérosol étant **caractérisé en ce que** le dispositif de commande est configuré pour amener l'ensemble indicateur à indiquer que le dispositif a cessé de fonctionner ou est sur le point de cesser de fonctionner dans une période de temps prédéterminée après que la bobine a commencé à chauffer la matière génératrice d'aérosol ;
dans lequel le dispositif comprend en outre une interface d'entrée (112), et le dispositif de commande est configuré pour amener la bobine à chauffer le suscepteur en réponse à une entrée reçue de l'interface d'entrée ;
dans lequel l'interface d'entrée est un bouton ;
dans lequel l'ensemble indicateur comprend un composant visuel comprenant une ou plusieurs LED (214) configurées pour fournir une indication visuelle selon laquelle le dispositif est prêt à être utilisé ;
dans lequel l'ensemble indicateur comprend un composant haptique configuré pour fournir un retour haptique afin d'indiquer que le dispositif est prêt à être utilisé ;
dans lequel l'ensemble indicateur est configuré pour fournir différents motifs de retour haptique pour indiquer le temps restant jusqu'à ce que le dispositif cesse de fonctionner à différents moments ;
dans lequel l'ensemble indicateur est configuré pour amener les une ou plusieurs LED du composant visuel à fournir une indication particulière pour indiquer que le dispositif est sur le point de cesser de fonctionner qui est différente d'une indication visuelle précédente.

2. Dispositif (100) de fourniture d'aérosol selon la revendication 1, dans lequel le dispositif de commande (302) est configuré pour amener l'ensemble indicateur (306) à indiquer que le dispositif est prêt à être utilisé dans une période de temps prédéterminée après avoir amené la bobine (124, 126) à chauffer le suscepteur (132) ou après qu'une température prédéterminée a été atteinte dans le dispositif, dans lequel la période de temps prédéterminée est inférieure à environ 30 secondes.

3. Dispositif (100) de fourniture d'aérosol selon la revendication 2, dans lequel la période de temps prédéterminée est inférieure à environ 15 secondes après avoir amené la bobine (124, 126) à chauffer le suscepteur (132).

4. Dispositif (100) de fourniture d'aérosol selon l'une quelconque des revendications 1 à 3, dans lequel le dispositif est configuré pour fonctionner dans l'un d'un premier mode et d'un deuxième mode, et lorsque le dispositif est utilisé dans le deuxième mode, la bobine est configurée pour amener la matière génératrice d'aérosol à être chauffée à une température plus élevée que lorsque le dispositif fonctionne dans le premier mode, et dans lequel la période de temps prédéterminée est une première durée prédéterminée lorsque le dispositif fonctionne dans le premier mode et une deuxième durée prédéterminée lorsque le dispositif fonctionne dans le deuxième mode, la deuxième durée prédéterminée étant différente de la première durée prédéterminée.

5. Dispositif (100) de fourniture d'aérosol selon l'une quelconque des revendications 1 à 4, dans lequel le dispositif est configuré pour fonctionner dans l'un d'un premier mode et d'un deuxième mode, et lorsque le dispositif est utilisé dans le deuxième mode, la bobine est configurée pour amener le suscepteur (132) à être chauffé à une température plus élevée que lorsque le dispositif fonctionne dans le premier mode, et dans lequel la période de temps prédéterminée est une première durée prédéterminée lorsque le dispositif fonctionne dans le premier mode et une deuxième durée prédéterminée lorsque le dispositif fonctionne dans le deuxième mode, la deuxième durée prédéterminée étant inférieure à la première durée prédéterminée.

6. Dispositif (100) de fourniture d'aérosol selon l'une quelconque des revendications 1 à 5, dans lequel l'entrée comprend un signal indiquant que le bouton a été relâché.

7. Dispositif (100) de fourniture d'aérosol selon la revendication 6, dans lequel l'entrée comprend en outre un signal indiquant une durée pendant laquelle le bouton a été enfoncé et le dispositif de commande (302) est configuré pour amener la bobine (124, 126) à chauffer le suscepteur (132) en réponse à :
la réception du signal indiquant que le bouton a été relâché ; et
la détermination que la durée pendant laquelle le bouton a été enfoncé est supérieure ou égale à une période de temps seuil.

8. Dispositif (100) de fourniture d'aérosol selon l'une quelconque des revendications 1 à 7, dans lequel le dispositif comprend en outre un détecteur de bouffée pour détecter quand un utilisateur prend une bouffée sur le dispositif, et le dispositif de commande (302) est configuré pour amener la bobine à chauffer le suscepteur (132) en réponse au fait que le détecteur détecte quand l'utilisateur prend une bouffée sur le dispositif.

9. Dispositif (100) de fourniture d'aérosol selon l'une quelconque des revendications 1 à 8, dans lequel l'ensemble indicateur (306) comprend un indicateur sonore configuré pour émettre un son pour indiquer que le dispositif est prêt à être utilisé.

10. Dispositif (100) de fourniture d'aérosol selon l'une quelconque des revendications 1 à 9, dans lequel la bobine est une première bobine (124), et le dispositif comprend en outre une deuxième bobine (126) pour chauffer le suscepteur (132), et dans lequel :
la première bobine est adjacente à la deuxième bobine dans une direction le long d'un axe longitudinal du dispositif ;
le dispositif de commande (302) est configuré pour amener la deuxième bobine à chauffer le suscepteur après avoir amené l'ensemble indicateur (306) à indiquer que le dispositif est prêt à être utilisé ; et
lors de l'utilisation, l'aérosol est aspiré le long d'un canal d'écoulement du dispositif vers une extrémité proximale du dispositif, et la première bobine est agencée plus près de l'extrémité proximale du dispositif que la deuxième bobine.

11. Dispositif (100) de fourniture d'aérosol selon l'une quelconque des revendications 1 à 10, dans lequel l'ensemble indicateur (306) est configuré pour fournir une indication de retour haptique selon laquelle la bobine d'induction (124, 126) a commencé à générer le champ magnétique variable.

12. Dispositif (100) de fourniture d'aérosol selon l'une quelconque des revendications 1 à 11, dans lequel chacune de la pluralité de LED (214) est configurée pour être éteinte lorsque le dispositif a cessé de fonctionner.

13. Système, comprenant :
le dispositif (100) selon l'une quelconque des revendications 1 à 12, comprenant en outre l'article (110), dans lequel le suscepteur est configuré pour générer de la chaleur en présence d'un champ magnétique variable.

14. Procédé de fonctionnement d'un dispositif (100) de fourniture d'aérosol, comprenant :
le fait d'amener une bobine d'induction (124, 126) du dispositif de fourniture d'aérosol à commencer à chauffer un suscepteur (132) pouvant être chauffé par la bobine d'induction pour chauffer une matière génératrice d'aérosol d'un article (110) reçu par une zone de chauffage et comprenant le suscepteur et la matière génératrice d'aérosol, l'article étant inséré à travers une ouverture (104) dans une extrémité du dispositif, et l'article pouvant être retiré de la zone de chauffage après utilisation ; dans lequel la bobine d'induction est hélicoïdale et entoure au moins une partie de la zone de chauffage ;
le fait d'amener un ensemble indicateur (306) du dispositif de fourniture d'aérosol à indiquer que le dispositif a cessé de fonctionner ou est sur le point de cesser de fonctionner dans une période de temps prédéterminée après avoir amené la bobine à commencer à chauffer la matière génératrice d'aérosol ;
le fait d'amener la bobine à chauffer le suscepteur en réponse à une entrée reçue d'une interface d'entrée (112) ; dans lequel l'interface d'entrée est un bouton ;
la fourniture, par un composant visuel de l'ensemble indicateur comprenant une ou plusieurs LED (214), d'une indication visuelle selon laquelle le dispositif est prêt à être utilisé ;
la fourniture, par un composant haptique de l'ensemble indicateur, d'un retour haptique pour indiquer que le dispositif est prêt à être utilisé ;
la fourniture, par l'ensemble indicateur, de différents motifs de retour haptique pour indiquer le temps restant jusqu'à ce que le dispositif cesse de fonctionner à différents moments ; et
le fait d'amener les une ou plusieurs LED du composant visuel de l'ensemble indicateur à fournir une indication particulière pour indiquer que le dispositif est sur le point de cesser de fonctionner qui est différente d'une indication visuelle précédente.

15. Procédé selon la revendication 14, comprenant :
le fait d'amener un ensemble indicateur (306) du dispositif (100) de fourniture d'aérosol à indiquer que le dispositif est prêt à être utilisé dans une période de temps prédéterminée après avoir amené la bobine (124, 126) à chauffer le suscepteur ou après qu'une température prédéterminée a été atteinte dans le dispositif, dans lequel la période de temps prédéterminée est inférieure à environ 30 secondes.
